# EUROPÄISCHE PATENTSCHRIFT

(11) **EP 2 990 015 B1**
(45) Veröffentlichungstag und Bekanntmachung des Hinweises auf die Patenterteilung: **07.02.2018**
(21) Anmeldenummer: 14002939.8
(22) Anmeldetag: 25.08.2014
(51) Int. Cl.: A61F 9/008

(54) **Ophthalmologisches Laserbehandlungssystem**
Ophthalmologic laser treatment system
Système de traitement laser ophtalmologique

(43) Veröffentlichungstag der Anmeldung: 02.03.2016
(73) Patentinhaber: Ziemer Ophthalmic Systems AG, 2562 Port (CH)
(72) Erfinder: Rathjen, Christian, 28197 Bremen (DE); Hertzberg, Joachim, 3604 Thun (CH)
(74) Vertreter: Rentsch Partner AG

(56) Entgegenhaltungen:
- WO-A2-2004/026198
- US-A1- 2013 158 531
- US-A1- 2014 104 600

## Beschreibung

### Technisches Gebiet

Die vorliegende Erfindung betrifft ein ophthalmologisches Laserbehandlungssystem. Die vorliegende Erfindung betrifft insbesondere ein ophthalmologisches Laserbehandlungssystem, das eine Laserquelle zur Erzeugung von Laserstrahlung, einen Lichtprojektor zur Fokussierung der Laserstrahlung auf einen Fokus und ein Scannersystem zum Bewegen des Fokus entlang einer Bearbeitungslinie umfasst.

### Stand der Technik

Bei der Behandlung von Augengewebe, beispielsweise zur refraktiven Korrektur der Hornhaut oder Linse, werden ophthalmologische Laserbehandlungssysteme eingesetzt, welche Laserstrahlung, insbesondere gepulste Laserstrahlung, fokussiert entlang einer Bearbeitungslinie projizieren, um so Gewebe aufzulösen oder Schnitte im Gewebe durchzuführen. Vorbereitend wird die Geometrie und Topologie des zu behandelnden Auges und seiner Strukturen erfasst und die geplante Bearbeitung mittels Bearbeitungsdaten definiert. Da die eigentliche Behandlung am lebenden Patienten durchgeführt wird, können jedoch Veränderungen und Positionsänderungen von Strukturen des Auges während der Behandlung nicht ausgeschlossen werden. Beispielsweise können sich intraoculare Strukturen verschieben. Beispielsweise können sich die Iris oder der hintere Kapselsack während der Behandlung verschieben. Überdies kann auch trotz fixierenden Patientenschnittstellen eine Bewegung des gesamten Auges nicht völlig ausgeschlossen werden. Es besteht daher die Gefahr, dass unerwünschte Stellen im Auge bearbeitet werden oder der Fokus zu nahe an solche Stellen geführt wird, so dass falsche Gewebebereiche oder Strukturen im Auge durch den gepulsten Laserstrahl bearbeitet und/oder zu stark belastet werden, so dass Schäden durch photochemische, photothermische und/oder photoakustische Effekte entstehen.

Die US 2014/104600 beschreibt ein Verfahren zum Bestimmen der Fokusposition eines Laserstrahls in einem ophthalmologischen Laserprojektionssystem. Gemäss US 2014/104600 werden mit dem Laserstrahl entlang einer Abtastbahn Messmarkierungen überfahren, die auf einer Referenzfläche angebracht sind. Dabei wird ein Messsignal erfasst, das beim Überfahren der Messmarkierungen durch Reflexion oder Absorption des Laserstrahls erzeugt wird und Signalflanken aufweist, die beim Überfahren von Kanten der Messmarkierungen entstehen. Die Fokusposition wird dann abhängig von Zeitwerten von mindestens einer Signalflanke ermittelt.

Die WO 2004/026198 beschreibt eine Vorrichtung zum Messen eines optischen Durchbruchs in einem Gewebe. Dazu ist gemäss WO 2004/026198 ein konfokales Mikroskop vorgesehen, das nach Art eines Laserscanning-Mikroskops arbeitet. Es ist eine Scannvorrichtung vorgesehen, die synchron sowohl die laterale Lage des Spotbilds, von dem reflektierte Strahlung zu einem Photomultiplier geleitet wird, als auch des Fokuspunktes eines Laserbehandlungsstrahls 2 verändert. Gemäss WO 2004/026198 können laterale Verschiebungen des Spots innerhalb der Objektebene gegenüber dem Fokuspunkt nicht eingestellt werden. Die WO 2004/026198 beschreibt eine alternative Lösung mit zwei unabhängigen Scanneinrichtungen, bei welcher der Behandlungslaser über einen eigenen Behandlungsscanner verfügt, der unabhängig von der Scanneinrichtung des konfokalen Mikroskops betätigbar ist. Diese aufwendigere Bauweise gestattet es, dass das konfokale Mikroskop aus dem Auge Strahlung an Punkten aufnehmen kann, die völlig unabhängig von der Fokuslage des Behandlungsstrahls gewählt werden können und nicht mit der Fokuslage des Behandlungsstrahls synchronisiert sind.

Die US 2013/158531 beschreibt eine Bildverarbeitungsvorrichtung für SD-OCT (Spectral Domain Optical Coherence Tomographie) während chirurgischen Laserkataraktverfahren. Gemäss US 2013/158531 wird ein SD-OCT System zur Bilderzeugung eines photobearbeiteten Gebiets verwendet. Aufgrund des erzeugten Bilds werden Feedback-Signale über Entwicklung, Ort und Ausdehnung des photobearbeiteten Gebiets erzeugt und darauf basierend alternative Bearbeitungswege eingeleitet.

### Darstellung der Erfindung

Es ist eine Aufgabe der vorliegenden Erfindung ein ophthalmologisches Laserbehandlungssystem vorzuschlagen, welches zumindest einige Nachteile des Stands der Technik nicht aufweist. Es ist insbesondere eine Aufgabe der vorliegenden Erfindung ein ophthalmologisches Laserbehandlungssystem vorzuschlagen, welches ermöglicht, Positionsänderungen von Strukturen im Auge während der Laserbehandlung des Auges zu erkennen.

Gemäss der vorliegenden Erfindung werden diese Ziele durch die Merkmale des unabhängigen Anspruchs erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass ein ophthalmologisches Laserbehandlungssystem mit einer Laserquelle zur Erzeugung von Laserstrahlung, einem Lichtprojektor zur Fokussierung der Laserstrahlung auf einen Fokus, und einem Scannersystem zum Bewegen des Fokus entlang einer Bearbeitungslinie überdies ein Überwachungssystem aufweist, das einen Lichtdetektor umfasst und eingerichtet ist, über einen Lichtweg einen sich dem Fokus mitbewegenden Überwachungsbereich zu überwachen. Die Überwachung eines sich mit dem Scannen entlang der Bearbeitungslinie mit dem Fokus mitbewegenden Bereichs ermöglicht die gezielte Detektion von Positionsänderungen von Strukturen im Auge oder des gesamten Auges relativ zum Laserbehandlungssystem während der Laserbehandlung des Auges. Dadurch können Augenstrukturen oder andere Objekte, wie beispielsweise Implantate, die sich unerwartet in den Bearbeitungsbereich, also auf die Bearbeitungslinie oder in die Nähe des Fokus respektive der Bearbeitungslinie verschieben, detektiert werden, bevor sie durch fokussierte Laserstrahlung, insbesondere durch einen fokussierten Laserpuls, getroffen werden oder zu nahe in den umgebenden Bereich der fokussierten Laserstrahlung, insbesondere in den umgebenden Bereich eines fokussierten Laserpulses, geraten.

Gegenüber bildgebenden und bildverarbeitenden Verfahren und Systemen, bei denen in der Regel das gesamte einsehbare Auge mit einer Kamera in der Aufsicht erfasst wird und das erfasst Aufsichtsbild aufwendig mit einem Bildverarbeitungsprozessor und Bildverarbeitungsalgorithmen verarbeitet wird, weist das vorliegende Überwachungssystem den Vorteil auf, dass durch eine optomechanische Kopplung und Nachführung des Überwachungsbereichs auf aufwendige Verarbeitungselektronik, Prozessoren und Algorithmen verzichtet werden kann, wodurch höhere Verarbeitungsgeschwindigkeiten mit geringerem Systemaufwand erzielbar sind. Die bei den bildgebenden und bildverarbeitenden Systemen notwendige Detektierung von Strukturen im Auge aus den erfassten Bilddaten entfällt durch die optomechanische Kopplung und Nachführung des Überwachungsbereichs und die Überwachung mittels eines vergleichsweise einfachen Lichtdetektors, der ohne dazu erforderliche Bildverarbeitung direkt Detektionssignale zur Detektierung von Strukturen respektive Strukturgrenzen im Überwachungsbereich liefert. Überdies können Probleme und Fehlverhalten in der aktiven Nachführung bildverarbeitender Systeme ausgeschlossen werden, die durch Softwarefehler, Hardwarefehler und Kalibrierungsfehler verursacht werden. Es erübrigen sich auch aufwendige Verfahrensschritte zum Verifizieren und Validieren bildverarbeitender Systeme unter verschiedensten Betriebsmodi und Bedingungen.

Je nach Ausführungsvariante ist das Überwachungssystem eingerichtet, über den Lichtweg einen sich optisch oder optomechanisch dem Fokus mitbewegenden Überwachungsbereich zu überwachen. Das heisst, der Überwachungsbereich ist optisch oder optomechanisch mit dem sich bewegenden Fokus gekoppelt, wie nachfolgend erläutert wird.

In einer Ausführungsvariante ist das Überwachungssystem eingerichtet, über den Lichtweg einen Überwachungsbereich zu überwachen, der sich mit fester geometrischer Zuordnung zum Fokus mit dem Fokus mitbewegt. Bei unveränderter Fokustiefe, ist die feste geometrische Zuordnung beispielsweise durch eine feste Distanz zwischen dem Fokus und dem Überwachungsbereich definiert, beispielsweise eine Distanz zwischen dem Fokus und einem Schnittpunkt des Lichtwegs mit einer durch den Fokus gelegten Fokalfläche.

Vorzugsweise ist der Lichtweg durch den Lichtprojektor zum Lichtdetektor geführt. In einer Ausführungsvariante ist der Lichtweg entlang einer Detektionsachse ausgerichtet, die einen definierten Ausrichtungswinkel zur optischen Achse z des Lichtprojektors aufweist. Der Ausrichtungswinkel bestimmt eine weitere feste geometrische Zuordnung von Fokus und Überwachungsbereich, wenn die Laserstrahlung nicht von der optischen Achse des Lichtprojektors abgelenkt wird, dabei bleibt der Ausrichtungswinkel auch bei unterschiedlicher Fokustiefe unverändert.

In einer weiteren Ausführungsvariante ist der Ausrichtungswinkel der Detektionsachse zur optischen Achse des Lichtprojektors einstellbar. Somit kann die relative Lage des Überwachungsbereichs bezüglich der optischen Achse des Lichtprojektors flexibel eingestellt werden.

In einer Ausführungsvariante umfasst das Scannersystem eine Antriebsvorrichtung zum Bewegen des Lichtprojektors entlang der Bearbeitungslinie, und der Lichtdetektor ist fest mit dem Lichtprojektor verbunden. In dieser Ausführungsvariante ist der Überwachungsbereich somit optomechanisch mit dem sich bewegenden Fokus gekoppelt und der Überwachungsbereich bewegt sich optomechanisch mit dem Fokus.

In einer Ausführungsvariante umfasst das Scannersystem mindestens einen beweglichen Spiegel zum Scannen des Augengewebes mit fokussierter Laserstrahlung entlang der Bearbeitungslinie. Der Lichtdetektor ist bezüglich eines von der Laserquelle zum Lichtprojektor verlaufenden Strahlengangs dem Scannersystem vorgeschaltet und der Lichtweg ist über den mindestens einen Spiegel des Scannersystems zum Lichtdetektor geführt. In dieser Ausführungsvariante ist der Überwachungsbereich somit optisch mit dem sich bewegenden Fokus gekoppelt und der Überwachungsbereich bewegt sich optisch mit dem Fokus. Die relative Lage des Überwachungsbereichs bezüglich der optischen Achse des Lichtprojektors wird beispielsweise durch Einstellung des Ausrichtungswinkels der Detektionsachse des Lichtdetektors zum Spiegel des Scannersystems bestimmt.

In einer weiteren Ausführungsvariante ist das Überwachungssystem eingerichtet, eine Tiefenüberwachung in einem in Richtung des Lichtwegs ausgedehnten Überwachungsbereich auszuführen.

In verschiedenen Ausführungsvarianten umfasst das Überwachungssystem ein interferometrisches Detektionssystem, ein auf axialer chromatischer Aberration mit spektraler Auswertung basierendes Detektionssystem, ein konfokales Detektionssystem, und/oder ein triangulierendes Detektionssystem.

In einer Ausführungsvariante umfasst das Überwachungssystem lichtempfindliche Elemente, die eingerichtet sind, Helligkeitswerte und/oder Spektralbereiche zu detektieren.

In einer Ausführungsvariante umfasst das Überwachungssystem eine Lichtquelle zur (aktiven) Beleuchtung des Überwachungsbereichs.

In einer Ausführungsvariante ist das Überwachungssystem eingerichtet, über den Lichtweg einen sich mit dem Fokus mitbewegenden und in Bewegungsrichtung des Fokus dem Fokus vorgelagerten, noch nicht durch Laserstrahlung bearbeiteten Überwachungsbereich zu überwachen.

In einer Ausführungsvariante ist das Überwachungssystem eingerichtet, mehrere sich mit dem Fokus mitbewegende Überwachungsbereiche über mehrere unterschiedliche Lichtwege zu überwachen. Die Lichtwege schneiden eine durch den Fokus gelegte Fokalfläche beispielsweise jeweils an einem anderen Schnittpunkt mit fester geometrischer Zuordnung zum Fokus. Je nach Ausführungsvariante umfasst das Überwachungssystem mehrere Lichtdetektoren, die jeweils einem unterschiedlichen Lichtweg zugeordnet sind, oder einen gemeinsamen Lichtdetektor, dem die verschiedenen Lichtwege zur Erzeugung eines Komposit-Signals zugeführt werden.

In einer Ausführungsvariante umfasst das Laserbehandlungssystem einen Datenspeicher mit Geometriedaten von Augenstrukturen, die vor der Behandlung erfasst wurden, und das Überwachungssystem umfasst eine mit dem Lichtdetektor gekoppelte Verarbeitungseinheit, die eingerichtet ist, abhängig von den Geometriedaten und von Detektionssignalen des Lichtdetektors im Überwachungsbereich örtliche Abweichungen von den durch die Geometriedaten definierten Augenstrukturen zu detektieren.

In einer Ausführungsvariante ist die Verarbeitungseinheit eingerichtet, bei einer Detektierung von Abweichungen Steuersignale für das Laserbehandlungssystem zu erzeugen, wobei die Steuersignale einen Befehl zum Deaktivieren der Laserquelle und/oder zum Unterbrechen der Projektion von Laserstrahlung umfassen.

In einer Ausführungsvariante umfasst das Überwachungssystem ein in den Lichtweg eingekoppeltes, dem Lichtdetektor vorgeschaltetes Fokussiersystem. Das Fokussiersystem ist eingerichtet, die Tiefenlage des Überwachungsbereichs in Richtung der optischen Achse des Lichtprojektors einzustellen.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1a:: zeigt schematisch ein ophthalmologisches Laserbehandlungssystem mit einem Überwachungssystem, das mittels eines Lichtdetektors einen im Querschnitt dargestellten Überwachungsbereich überwacht, der sich mit dem Fokus mitbewegt, auf den das Laserbehandlungssystem Laserstrahlung fokussiert.
- Figur 1b:: zeigt schematisch einen Überwachungsbereich in der Aufsicht, der sich mit einer festen geometrischen Zuordnung zum Fokus mit dem Fokus entlang der Bearbeitungslinie mitbewegt.
- Figur 2:: zeigt schematisch ein ophthalmologisches Laserbehandlungssystem mit einem durch eine Antriebsvorrichtung bewegten Lichtprojektor zur Fokussierung der Laserstrahlung und einem mitbewegten Lichtdetektor, der einen sich mit dem Fokus mitbewegenden Überwachungsbereich überwacht.
- Figur 3:: zeigt schematisch ein ophthalmologisches Laserbehandlungssystem mit einem beweglichen Spiegel zum Scannen des Augengewebes mit fokussierter Laserstrahlung und einem Überwachungssystem, das über einen über den Spiegel geführten Lichtweg einen sich mit dem Fokus mitbewegenden Überwachungsbereich überwacht, der im Querschnitt dargestellt ist.

### Wege zur Ausführung der Erfindung

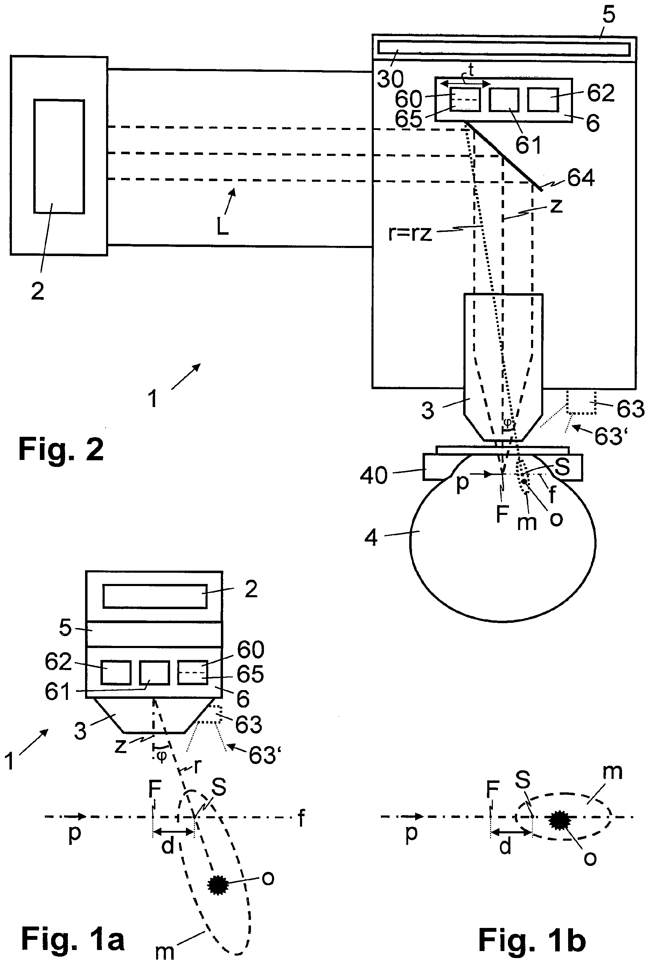

In den Figuren 1a, 2 und 3 bezieht sich das Bezugszeichen 1 auf ein ophthalmologisches Laserbehandlungssystem, das eine Laserquelle 2 zur Erzeugung von Laserstrahlung L, einen Lichtprojektor 3 zur Fokussierung der Laserstrahlung L auf einen Fokus F, und ein Scannersystem 5 zum Bewegen des Fokus F entlang einer Bearbeitungslinie p umfasst. In einer Ausführungsvariante ist die Laserquelle 2 eingerichtet, gepulste Laserstrahlung zu erzeugen, und umfasst beispielsweise einen Femtolaser zur Erzeugung von Femtolaserpulsen L. Der Fachmann wird verstehen, dass ohne Abweichung vom Anspruchsgegenstand Laserquellen 2 zur Erzeugung von gepulster als auch von nicht gepulster Laserstrahlung respektive Laserstahlen einsetzbar sind. Das ophthalmologische Laserbehandlungssystem umfasst in der Regel ein nicht dargestelltes optisches Übertragungssystem, das eingerichtet ist den gepulsten Laserstrahl so von der Laserquelle 2 zum Lichtprojektor 3 zu übertragen, dass dieser im Wesentlichen kollimiert oder zumindest annähend kollimiert beim Lichtprojektor 3 eintritt. Es sei hier festgehalten, dass sich das hier vorgestellte Verfahren respektive System in für den Fachmann bekannte Weise auch auf nicht kollimierte Strahlengänge übertragen respektive anwenden lässt.

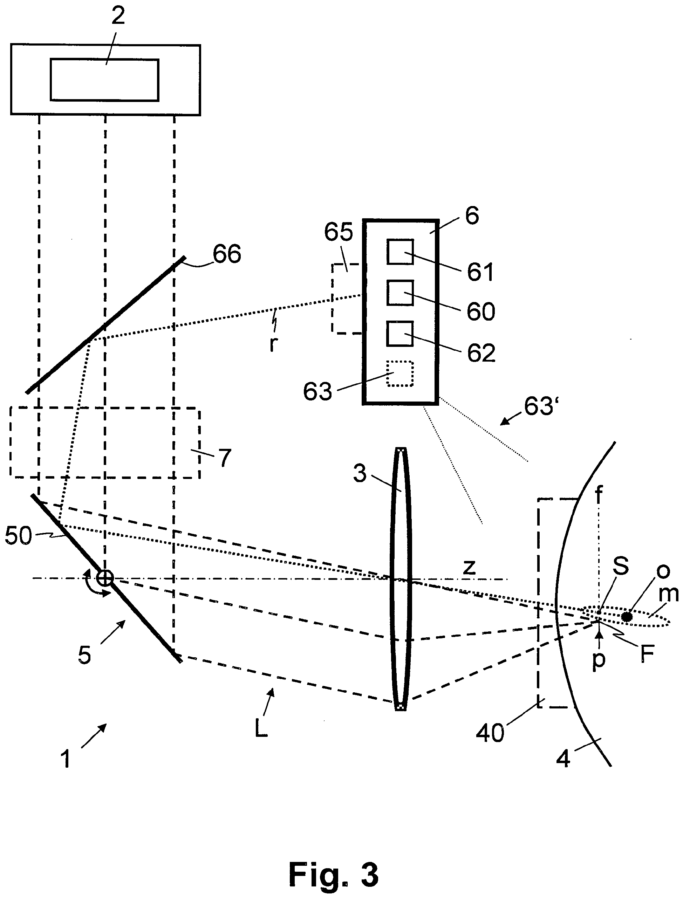

Wie in den Figuren 2 und 3 schematisch dargestellt ist, umfasst das ophthalmologische Laserbehandlungssystem 1 optional ein Patienteninterface 40, mittels dessen das Auge des Patienten während der Behandlung fixiert wird, beispielsweise ein Saugring mit oder ohne Kontaktkörper.

In der Ausführungsvariante der Figur 2 umfasst das Scannersystem 5 eine mit dem Lichtprojektor 3 gekoppelte Antriebsvorrichtung 30 zum Bewegen des Lichtprojektors 3 entlang einer Bearbeitungslinie p, beispielsweise ein elektrischer Motor. Durch die Bewegung des Lichtprojektors 3 wird auch der Fokus F mitbewegt, auf den die Laserstrahlung L (respektive die Laserpulse) durch den Lichtprojektor 3 fokussiert wird.

In der Ausführungsvariante der Figur 3 umfasst das Scannersystem 5 einen oder mehrere bewegliche Spiegel 50, z.B. drehbar um eine oder mehrere Spiegelachsen, zum Scannen des Augengewebes 4 mit fokussierter Laserstrahlung (respektive mit fokussierten Laserpulsen) entlang einer Bearbeitungslinie p. Die Laserstrahlung L (respektive der gepulste Laserstrahl) wird durch den/die Spiegel 50 so abgelenkt, dass der Fokus F, auf den sie vom Lichtprojektor 3 fokussiert wird, sich entlang der Bearbeitungslinie p bewegt.

In beiden Ausführungsvarianten umfasst das Laserbehandlungssystem 1 überdies ein Überwachungssystem 6, das eingerichtet ist, einen Überwachungsbereich m zu überwachen, der sich mit fester geometrischer Zuordnung zum Fokus F mit dem Fokus F mitbewegt. Das Überwachungssystem 6 umfasst einen Lichtdetektor 60, eine Verarbeitungseinheit 61 und einen Datenspeicher 62. Optional umfasst das Überwachungssystem 6 zudem eine Lichtquelle 63 zur Beleuchtung des Überwachungsbereichs m, beispielsweise eine oder mehrere LED. Neben einer externen Beleuchtung kann auch entlang des Lichtwegs r beleuchtet werden. Der Lichtdetektor 60 umfasst lichtempfindliche Elemente, beispielsweise Photodioden oder andere Lichtsensoren, die eingerichtet sind, Helligkeitswerte und/oder Spektralbereiche zu detektieren. Helligkeitsempfindliche Photodioden werden zur Detektion von Strukturübergängen respektive -grenzen eingesetzt, zum Beispiel von der schwarzen Pupille zur hellen Iris. Spektralempfindliche Photodioden werden zur Detektion von spezifischen Strukturen mit vorgegebenen Farbspektren eingesetzt, beispielsweise ein definierter Farbspektrumsbereich zur Detektion der Iris. Das Überwachungssystem 6 ist eingerichtet, dem Lichtdetektor 60 über einen Lichtweg r Licht vom Überwachungsbereich m zuzuführen, das von Augengewebe 4 und anderen Objekten o im Überwachungsbereich m aufgrund von Umgebungslicht, von eingestrahlter Laserstrahlung L, und/oder von Beleuchtungslicht 63' der Lichtquelle 63 reflektiert wird.

Die Verarbeitungseinheit 61 ist mit dem Lichtdetektor 60 über Signalleitungen verbunden und eingerichtet, Detektionssignale vom Lichtdetektor 60 zu empfangen und auszuwerten. Die Verarbeitungseinheit 61 ist über Datenleitungen mit dem Datenspeicher 62 verbunden und eingerichtet, im Datenspeicher 62 Geometriedaten von Augenstrukturen zu lesen und auszuwerten. In einer Variante ist die Verarbeitungseinheit 61 überdies eingerichtet, im Datenspeicher 62 Geometriedaten von Augenstrukturen zu speichern, die vor der Behandlung erfasst werden und beispielsweise über eine Datenleitung von einem internen oder externen Messsystem eingespeist werden. Die Geometriedaten umfassen Topologie, Form, Umriss, Grösse, Position und/oder Anordnung von Augenstrukturen wie Hornhaut, Iris, Pupille, Sklera, Limbus, Linse, Netzhaut und/oder Glaskörper, sowie andere Objekte o wie Implantate oder andere Fremdkörper im Augengewebe 4. Die Verarbeitungseinheit 61 ist eingerichtet, abhängig von den Geometriedaten und von Detektionssignalen des Lichtdetektors 60 zu detektieren, ob die vom Lichtdetektor 60 gelieferten Detektionssignale im Überwachungsbereich m örtliche Abweichungen von Strukturen im Auge anzeigen, die durch die Geometriedaten definiert werden. Dabei berücksichtigt die Verarbeitungseinheit 61 bloss Detektionssignale von Überwachungsbereichen m die noch nicht bearbeitet wurden, um Fehldetektionen zu vermeiden, die auf durch die Laserbearbeitung verursachten Veränderungen im Augengewebe 4 beruhen. Die Verarbeitungseinheit 61 ist eingerichtet, bei einer Detektierung von Abweichungen Steuersignale für das Laserbehandlungssystem 1 zu erzeugen, die Laserquelle 2 zu deaktivieren und/oder die Projektion von Laserstrahlung L (respektive von Laserpulsen) zu unterbrechen, wenn die Abweichungen eine unerwartete Lage von Strukturen entlang der Bearbeitungslinie p anzeigen. Die Verarbeitungseinheit 61 umfasst einen Schaltkreis, beispielsweise einen oder mehrere Prozessoren oder andere programmierbare Logikeinheiten, die eingerichtet sind, die durch die gespeicherten Geometriedaten im Überwachungsbereich m definierten Strukturen hinsichtlich ihrer Lage mit den vom Lichtdetektor 60 detektierten Strukturen respektive Strukturgrenzen zu vergleichen, um unerwünschte Positionsänderungen der Strukturen während der Behandlung zu detektieren und die Behandlung abzubrechen oder zumindest zu unterbrechen.

An dieser Stelle soll klar darauf hingewiesen werden, dass im Unterschied zu bildgebenden respektive bildverarbeitenden Verfahren oder Systemen die vom Lichtdetektor 60 gelieferten Detektionssignale keiner Bildverarbeitung bedürfen, sondern direkt mit ortsspezifischen Strukturangaben m verglichen werden, die von der Verarbeitungseinheit 61 aus den Geometriedaten für den Überwachungsbereich m entnommen werden. Abhängig von der Ausführung des Lichtdetektors 60 werden dabei durch die Detektionssignale gelieferte Helligkeitswerte und/oder Farbwerte (Spektralwerte) ortsspezifisch mit entsprechenden Werten aus den Geometriedaten verglichen.

In der Ausführungsvariante der Figur 2 ist der Lichtdetektor 60 fest mit dem Lichtprojektor 3 verbunden und wird bei der Bewegung des Lichtprojektors 3 entlang der Bearbeitungslinie p mitbewegt. Der Lichtdetektor 60 ist eingerichtet, den Überwachungsbereich m über einen Lichtweg r zu überwachen, der durch den Lichtprojektor 3 verläuft. Der Lichtweg r ist entlang einer Detektionsachse rz ausgerichtet, die einen definierten Ausrichtungswinkel ϕ zur optischen Achse z des Lichtprojektors 3 aufweist. Dadurch, dass der Lichtdetektor 60 zusammen mit dem Lichtprojektor 3 mitbewegt wird, definiert der Ausrichtungswinkel ϕ der Detektionsachse rz des Lichtdetektors 60 zur optischen Achse z des Lichtprojektors 3 eine feste geometrische Zuordnung des Überwachungsbereichs m zum Fokus F des Lichtprojektors 3, die auch bei der Bewegung des Lichtprojektors 3 respektive seines Fokus F erhalten bleibt. Im Spezialfall, wo der Ausrichtungswinkel ϕ Null beträgt, ist der Überwachungsbereich m konzentrisch zum Fokus F des Lichtprojektors 3 (und umgibt den Fokus F mit einem grösseren Radius als die Spotgrösse eines fokussierten Laserstrahls respektive Laserpulses). Ansonsten, verläuft der Lichtweg r entlang einer zur optischen Achse z des Lichtprojektors 3 mit Ausrichtungswinkel ϕ abgewinkelten Detektionsachse rz, wie in den Figuren 1a und 2 dargestellt ist. Bei unveränderter Tiefe des Fokus F in Richtung der optischen Achse z des Lichtprojektors 3 bleibt dabei auch die geometrische Zuordnung hinsichtlich der Distanz d des Überwachungsbereichs m zum Fokus F konstant, wobei die Distanz d in den Figuren 1a (Querschnitt) und 1b (Aufsicht) als Abstand des Fokus F zum Schnittpunkt S des Lichtwegs r mit der Fokalfläche f des Lichtprojektors 3 dargestellt ist. Die Figuren 1a, 1b, 2 und 3 illustrieren einen dem Fokus F in (auf der Bearbeitungslinie p mit einem Pfeil angedeuteten) Bearbeitungsrichtung vorgelagerten Überwachungsbereich m, der vorausschauend einen noch nicht bearbeiteten Bereich des Augengewebes 4 abdeckt, der vor dem aktuell bearbeiteten Gewebe liegt. Wie in der Figur 2 illustriert ist, wird der gepulste Laserstrahl von der Laserquelle 2 über einen Umlenkspiegel 64 zum beweglichen Lichtprojektor 3 geführt. Der Lichtweg r respektive die Detektionsachse rz führt durch eine Öffnung (oder eine spektral selektive Beschichtung oder einen kleinen eingebrachten Spiegel) im Umlenkspiegel 64 oder am Umlenkspiegel 64 vorbei zum Lichtdetektor 60. In einer Ausführungsvariante ist der Ausrichtungswinkel ϕ der Detektionsachse rz zur optischen Achse z des Lichtprojektors 3 einstellbar. Dazu ist die Position des Lichtdetektors 60 relativ zum Lichtprojektor 3 veränderbar, beispielsweise durch Verschieben des Lichtdetektors 60 entlang einer zur Projektionsachse z normal verlaufenden Translationsachse t, die in Richtung der Bearbeitungslinie p ausgerichtet ist. In einer weiteren Ausführungsvariante ist die Ausdehnung des Überwachungsbereichs m einstellbar, beispielsweise durch eine einstellbare Lochblende, die das dem Lichtdetektor 60 zugeführte Licht begrenzt.

In der Ausführungsvariante der Figur 3 führt der Lichtweg r durch den Lichtprojektor 3 und über den einen oder mehrere Spiegel 50 des Scannersystems 5 zum Lichtdetektor 60. Das heisst, der Lichtdetektor 60 ist im Strahlengang von der Laserquelle 2 zum Lichtprojektor 3 dem Scannersystem 5 vorgeschaltet (respektive im Strahlengang vom Lichtprojektor 3 zur Laserquelle dem Scannersystem 5 nachgeschaltet). Der Lichtdetektor 60 wird über ein optisches Element 66, beispielsweise ein halbdurchlässiger Spiegel respektive Strahlteiler, auf den Lichtweg r zum Überwachungsbereich m gekoppelt. Durch das Vorschalten des Lichtdetektors 60 vor das Scannersystem 5 und das Führen des Lichtwegs r über den/die Spiegel 50 des Scannersystems 5 wird bei der durch die Bewegung des/der Spiegel 50 bewirkten Ablenkung der Laserstrahlung (respektive Laserpulsen) und der damit erzielten Bewegung des Fokus F der fokussierten Laserstrahlung (respektive des gepulsten Laserstrahls) entlang der Bearbeitungslinie p auch der Überwachungsbereich m mit einer festen geometrischen Zuordnung zum Fokus mitbewegt. Mit anderen Worten, durch die scannende Bewegung des/der Spiegel 50 des Scannersystems wird der Lichtweg r synchron mit dem gepulsten Laserstrahl entlang der Bearbeitungslinie p gescannt, so dass der Überwachungsbereich m mit der Bewegung des Fokus F der projizierten Laserstrahlung (respektive Laserpulse) entlang der Bearbeitungslinie p mitgeführt wird.

In einer Variante des ophthalmologischen Laserbehandlungssystems 1, in der ein Divergenzmodulator 7 im Strahlengang von der Laserquelle 2 zum Lichtprojektor 3 angeordnet ist, ist der Lichtdetektor 60 dem Divergenzmodulator 7 vorgeschaltet, so dass durch Divergenzmodulation bewirkte Fokusänderungen der projizierten Laserstrahlung (respektive Laserpulse) entsprechend auch im Lichtweg r und damit für den Überwachungsbereich m vorgenommen werden. Dies gilt entsprechend auch für Fokusänderungen durch den Lichtprojektor 3, so dass die geometrische Zuordnung des Überwachungsbereichs m zum Fokus F auch bei Fokusverschiebungen in Projektionsrichtung erhalten bleibt und der Überwachungsbereich m auch in Projektionsrichtung mit fester geometrischer Zuordnung zum Fokus F mit dem Fokus F mitbewegt wird. Bei Anwendungen, bei denen eine Mitbewegung des Überwachungsbereichs m in Projektionsrichtung bei Varianten mit Divergenzmodulatoren 7 unerwünscht ist, wird der Lichtdetektor 60 dem Divergenzmodulator 7 nachgeschaltet.

In einer Ausführungsvariante ist die Tiefenlage des Überwachungsbereichs m in Richtung der optischen Achse z des Lichtprojektors 3 einstellbar. Dazu umfasst das Überwachungssystem 6 ein in den Lichtweg r dem Lichtdetektor 60 vorgeschaltetes Fokussiersystem 65, das eine oder mehrere verschiebbare Linsen aufweist.

In einer Ausführungsvariante ist das Überwachungssystem 6 eingerichtet, eine Tiefenüberwachung in einem in Projektionsrichtung respektive in Richtung des Lichtwegs r ausgedehnten Überwachungsbereich m auszuführen, insbesondere ein Überwachungsbereich m, der auch einen in Projektionsrichtung unterhalb der Fokalfläche f liegenden Bereich umfasst, wie in den Figuren 1a, 2 und 3 schematisch dargestellt ist. Dazu umfasst das Überwachungssystem 6 ein interferometrisches Detektionssystem, ein auf chromatischer Aberration basierendes Detektionssystem, ein konfokales Detektionssystem, und/oder ein triangulierendes Detektionssystem. Abhängig von der Ausführungsvariante weist das Überwachungssystem 6 ein tiefenauflösendes Detektionssystem zusätzlich zu einem Detektionssystem ohne Tiefenauflösung auf, beispielsweise Lichtdetektoren 60 mit den oben erwähnten Photodioden.

An dieser Stelle soll festgehalten werden, dass das Überwachungssystem 6 eingerichtet ist, über den Lichtweg r einen sich mit dem Fokus F mitbewegenden Überwachungsbereich m zu überwachen, der nicht nur Bewegungen des Fokus F in der Bewegungsrichtung auf der Bearbeitungslinie p in normal zur optischen Achse z des Lichtprojektors 3 verlaufenden x-/y-Richtung, sondern auch in z-Projektionsrichtung umfasst. Zur Positionierung eines sich mit dem Fokus F in z- oder Projektionsrichtung mitbewegenden Überwachungsbereichs m ist in einer Ausführungsvariante ein zusätzliches in den Lichtweg r eingefügtes, dem Lichtprojektor 3 vorgeschaltetes Fokussiermodul vorgesehen, mit dem die geometrische Zuordnung des Überwachungsbereichs m zum Fokus F für Fokusverschiebungen in Projektionsrichtung einstellbar ist.

An dieser Stelle soll überdies festgehalten werden, dass das Überwachungssystem 6 in weiteren Ausführungsvarianten mehrere Lichtdetektoren 60 respektive Detektionssysteme zur Überwachung mehrerer sich mit dem Fokus F mitbewegenden Überwachungsbereiche m umfasst. Dabei sind diese Lichtdetektoren 60 respektive Detektionssysteme eingerichtet, die verschiedenen Überwachungsbereiche m über mehrere unterschiedliche Lichtwege r zu überwachen, die eine durch den Fokus F gelegte Fokalfläche f jeweils an einem anderen Schnittpunkt S mit fester geometrischer Zuordnung zum Fokus F schneiden. In einer Ausführungsvariante werden die verschiedenen Lichtwege r dabei so ausgeführt, dass mehrere Überwachungsbereiche m, beispielsweise mittels diffraktiver Optiken, überwacht werden, deren Lichtwege dann alle zu einem gemeinsamen Lichtdetektor 60 geführt werden, wo daraus ein Komposit-Signal erzeugt wird. In einer weiteren Variante werden mehrere Lichtleiter vorgesehen. Die verschiedenen Überwachungsbereiche m sind in einem den Fokus F umgebenden Bereich angeordnet. Durch die Überwachung von mehreren gezielt ausgewählten und beschränkten Bereichen in der Nähe des Fokus F wird eine hohe Detektionsrate mit geringen Latenzzeiten erreicht, effizienter als dies beispielsweise bei bildgebenden Systemen möglich ist.

## Patentansprüche

1. Ophthalmologisches Laserbehandlungssystem (1), umfassend eine Laserquelle (2) zur Erzeugung von Laserstrahlung (L), einen Lichtprojektor (3) zur Fokussierung der Laserstrahlung (L) auf einen Fokus (F), ein Scannersystem (5) zum Bewegen des Fokus (F) entlang einer Bearbeitungslinie (p), und ein Überwachungssystem (6), das einen Lichtdetektor (60) umfasst, **dadurch gekennzeichnet, dass** das Überwachungssystem (6) eingerichtet ist, über einen entlang einer zur optischen Achse (z) des Lichtprojektors (3) abgewinkelten Detektionsachse (rz) verlaufenden Lichtweg (r) einen sich mit dem Fokus (F) mitbewegenden Überwachungsbereich (m) zu überwachen, um Objekte (o) zu detektieren, bevor sie durch fokussierte Laserstrahlung (L) getroffen werden.

2. Laserbehandlungssystem (1) nach Anspruch 1, **dadurch gekennzeichnet, dass** der Lichtweg (r) durch den Lichtprojektor (3) zum Lichtdetektor (60) geführt ist und entlang der Detektionsachse (rz) ausgerichtet ist, die einen definierten Ausrichtungswinkel (ϕ) zur optischen Achse (z) des Lichtprojektors (3) aufweist.

3. Laserbehandlungssystem (1) nach Anspruch 2, **dadurch gekennzeichnet, dass** der Ausrichtungswinkel (ϕ) der Detektionsachse (rz) zur optischen Achse (z) des Lichtprojektors (3) einstellbar ist.

4. Laserbehandlungssystem (1) nach einem der Ansprüche 1 bis 3, **dadurch gekennzeichnet, dass** das Scannersystem (5) eine Antriebsvorrichtung (30) zum Bewegen des Lichtprojektors (3) entlang der Bearbeitungslinie (p) umfasst, und dass der Lichtdetektor (60) fest mit dem Lichtprojektor (3) verbunden ist.

5. Laserbehandlungssystem (1) nach einem der Ansprüche 1 bis 3, **dadurch gekennzeichnet, dass** das Scannersystem (5) mindestens einen beweglichen Spiegel (50) zum Scannen des Augengewebes (4) mit fokussierter Laserstrahlung (L) entlang der Bearbeitungslinie (p) umfasst, dass der Lichtdetektor (60) bezüglich eines von der Laserquelle (2) zum Lichtprojektor (3) verlaufenden Strahlengangs dem Scannersystem (5) vorgeschaltet ist, und dass der Lichtweg (r) über den mindestens einen Spiegel (50) des Scannersystems (5) zum Lichtdetektor (60) geführt ist.

6. Laserbehandlungssystem (1) nach einem der Ansprüche 1 bis 5, **dadurch gekennzeichnet, dass** das Überwachungssystem (6) eingerichtet ist, eine Tiefenüberwachung in einem in Richtung des Lichtwegs (r) ausgedehnten Überwachungsbereich (m) auszuführen.

7. Laserbehandlungssystem (1) nach einem der Ansprüche 1 bis 6, **dadurch gekennzeichnet, dass** das Überwachungssystem (6) mindestens eines umfasst aus: ein interferometrisches Detektionssystem, ein auf axialer chromatischer Aberration mit spektraler Auswertung basierendes Detektionssystem, ein konfokales Detektionssystem, und ein triangulierendes Detektionssystem.

8. Laserbehandlungssystem (1) nach einem der Ansprüche 1 bis 7, **dadurch gekennzeichnet, dass** der Lichtdetektor (60) lichtempfindliche Elemente umfasst, die eingerichtet sind, mindestens eines aus Helligkeitswerte und Spektralbereiche zu detektieren.

9. Laserbehandlungssystem (1) nach einem der Ansprüche 1 bis 8, **dadurch gekennzeichnet, dass** das Überwachungssystem (6) eine Lichtquelle (63) zur Beleuchtung des Überwachungsbereichs (m) umfasst.

10. Laserbehandlungssystem (1) nach einem der Ansprüche 1 bis 9, **dadurch gekennzeichnet, dass** das Überwachungssystem (6) eingerichtet ist, über den Lichtweg (r) einen sich mit dem Fokus (F) mitbewegenden und in Bewegungsrichtung des Fokus (F) dem Fokus (F) vorgelagerten, noch nicht durch Laserstrahlung bearbeiteten Überwachungsbereich (m) zu überwachen.

11. Laserbehandlungssystem (1) nach einem der Ansprüche 1 bis 10, **dadurch gekennzeichnet, dass** das Überwachungssystem (6) eingerichtet ist, mehrere sich mit dem Fokus (F) mitbewegende Überwachungsbereiche (m) über mehrere unterschiedliche Lichtwege (r) zu überwachen, die eine durch den Fokus (F) gelegte Fokalfläche (f) jeweils an einem anderen Schnittpunkt (S) mit fester geometrischer Zuordnung zum Fokus (F) schneiden.

12. Laserbehandlungssystem (1) nach einem der Ansprüche 1 bis 11, **dadurch gekennzeichnet, dass** das Laserbehandlungssystem (1) einen Datenspeicher (62) mit Geometriedaten von vor der Behandlung erfassten Augenstrukturen umfasst, und dass das Überwachungssystem (6) eine mit dem Lichtdetektor (60) gekoppelte Verarbeitungseinheit (62) umfasst, die eingerichtet ist, abhängig von den Geometriedaten und von Detektionssignalen des Lichtdetektors (60) im Überwachungsbereich (m) örtliche Abweichungen von den durch die Geometriedaten definierten Augenstrukturen zu detektieren.

13. Laserbehandlungssystem (1) nach Anspruch 12, **dadurch gekennzeichnet, dass** die Verarbeitungseinheit (62) eingerichtet ist, bei einer Detektierung von Abweichungen Steuersignale für das Laserbehandlungssystem (1) zu erzeugen, wobei die Steuersignale mindestens einen Befehl umfassen aus: Deaktivieren der Laserquelle (2) und Unterbrechen der Projektion von Laserstrahlung (L).

14. Laserbehandlungssystem (1) nach einem der Ansprüche 1 bis 13, **dadurch gekennzeichnet, dass** das Überwachungssystem (6) eingerichtet ist, über den Lichtweg (r) einen Überwachungsbereich (m) zu überwachen, der sich mit fester geometrischer Zuordnung zum Fokus (F) mit dem Fokus (F) mitbewegt.

15. Laserbehandlungssystem (1) nach einem der Ansprüche 1 bis 14, **dadurch gekennzeichnet, dass** das Überwachungssystem (6) ein in den Lichtweg (r) eingekoppeltes, dem Lichtdetektor (60) vorgeschaltetes Fokussiersystem (65) umfasst.

## Claims

1. Ophthalmological laser treatment system (1), comprising a laser source (2) for producing laser radiation (L), a light projector (3) for focusing the laser radiation (L) onto a focus (F), a scanner system (5) for moving the focus (F) along a work line (p), and a monitoring system (6), which comprises a light detector (60), **characterized in that** the monitoring system (6) is configured to monitor, by way of a light path (r) extending along a detection axis (rz) angled in relation to the optical axis (z) of the light projector (3), a monitored region (m) moving together with the focus (F), in order to detect objects (o) before they are met by focused laser radiation (L).

2. Laser treatment system (1) according to Claim 1, **characterized in that** the light path (r) is guided through the light projector (3) to the light detector (60) and aligned along the detection axis (rz), which has a defined alignment angle (ϕ) in relation to the optical axis (z) of the light projector (3).

3. Laser treatment system (1) according to Claim 2, **characterized in that** the alignment angle (ϕ) of the detection axis (rz) is adjustable in relation to the optical axis (z) of the light projector (3).

4. Laser treatment system (1) according to one of Claims 1 to 3, **characterized in that** the scanner system (5) comprises a drive device (30) for moving the light projector (3) along the work line (p) and **in that** the light detector (60) is fixedly connected to the light projector (3).

5. Laser treatment system (1) according to one of Claims 1 to 3, **characterized in that** the scanner system (5) comprises at least one movable mirror (50) for scanning the eye tissue (4) with focussed laser radiation (L) along the work line (p), **in that** the light detector (60) is disposed upstream of the scanner system (5) in relation to a beam path extending from the laser source (2) to the light projector (3) and **in that** the light path (r) is guided to the light detector (60) via the at least one mirror (50) of the scanner system (5).

6. Laser treatment system (1) according to one of Claims 1 to 5, **characterized in that** the monitoring system (6) is configured to perform depth monitoring in a monitored region (m) extending in the direction of the light path (r).

7. Laser treatment system (1) according to one of Claims 1 to 6, **characterized in that** the monitoring system (6) comprises at least one of the following: an interferometric detection system, a detection system based on axial chromatic aberration with spectral evaluation, a confocal detection system and a triangulating detection system.

8. Laser treatment system (1) according to one of Claims 1 to 7, **characterized in that** the light detector (60) comprises light-sensitive elements, which are configured to detect at least one of brightness values and spectral regions.

9. Laser treatment system (1) according to one of Claims 1 to 8, **characterized in that** the monitoring system (6) comprises a light source (63) for illumination of the monitored region (m).

10. Laser treatment system (1) according to one of Claims 1 to 9, **characterized in that** the monitoring system (6) is configured to monitor, by way of the light path (r), a monitored region (m), which moves together with the focus (F) and is disposed upstream of the focus (F) in the movement direction of the focus (F) and not yet worked on by the laser radiation.

11. Laser treatment system (1) according to one of Claims 1 to 10, **characterized in that** the monitoring system (6) is configured to monitor a plurality of monitored regions (m) moving together with the focus (F) by way of a plurality of different light paths (r), which each intersect a focal surface (f) placed through the focus (F) at a different point of intersection (S) with a fixed geometric assignment to the focus (F).

12. Laser treatment system (1) according to one of Claims 1 to 11, **characterized in that** the laser treatment system (1) comprises a data storage medium (62) with geometry data of eye structures registered prior to the treatment and **in that** the monitoring system (6) comprises a processing unit (62) coupled to the light detector (60), which processing unit is configured to detect local deviations of the eye structures defined by the geometry data in the monitored region (m) as a function of the geometry data and the detection signals of the light detector (60) .

13. Laser treatment system (1) according to Claim 12, **characterized in that** the processing unit (62) is configured to produce control signals for the laser treatment system (1) when deviations are detected, wherein the control signals comprise at least one command from the following: deactivating the laser source (2) and interrupting the projection of laser radiation (L).

14. Laser treatment system (1) according to one of Claims 1 to 13, **characterized in that** the monitoring system (6) is configured to monitor, by way of the light path (r), a monitored region (m) which moves together with the focus (F) with a fixed geometric assignment to the focus (F).

15. Laser treatment system (1) according to one of Claims 1 to 14, **characterized in that** the monitoring system (6) comprises a focusing system (65) which is coupled into the light path (r) and disposed upstream of the light detector (60).

## Revendications

1. Système de traitement laser ophtalmologique (1), comprenant une source laser (2) destinée à générer un rayonnement laser (L), un projecteur de lumière (3) destiné à focaliser le rayonnement laser (L) sur un foyer (F), un système de balayage (5) destiné à déplacer le foyer (F) le long d'une ligne de traitement (p) et un système de surveillance (6) qui comprend un détecteur de lumière (60),
**caractérisé en ce que** le système de surveillance (6) est conçu pour surveiller une zone de surveillance (m) se déplaçant en association avec le foyer (F) sur un chemin optique (r) s'étendant le long d'un axe de détection (rz) formant un angle par rapport à un axe optique (z) du projecteur de lumière (3) afin de détecter des objets (o) avant qu'ils soient exposés au rayonnement laser (L) focalisé.

2. Système de traitement laser (1) selon la revendication 1, **caractérisé en ce que** le chemin optique (r) est guidé par l'intermédiaire du projecteur de lumière (3) vers le détecteur de lumière (60) et **en ce qu'**il est orienté le long de l'axe de détection (rz) qui présente un angle d'orientation (ϕ) défini par rapport à l'axe optique (z) du projecteur de lumière (3) .

3. Système de traitement laser (1) selon la revendication 2, **caractérisé en ce que** l'angle d'orientation (ϕ) de l'axe de détection (rz) par rapport à l'axe optique (z) du projecteur de lumière (3) est réglable.

4. Système de traitement laser (1) selon l'une quelconque des revendications 1 à 3, **caractérisé en ce que** le système de balayage (5) comprend un dispositif d'entrainement (30) destiné à déplacer le projecteur de lumière (3) le long de la ligne de traitement (p), et **en ce que** le détecteur de lumière (60) est relié de manière rigide au projecteur de lumière (3).

5. Système de traitement laser (1) selon l'une quelconque des revendications 1 à 3, **caractérisé en ce que** le système de balayage (5) comprend au moins un miroir mobile (50) destiné à balayer le tissu oculaire (4) avec un rayonnement laser (L) focalisé le long de la ligne de traitement (p), **en ce que** le détecteur de lumière (60) est disposé en amont du système de balayage (5) par rapport à un trajet de faisceau s'étendant de la source laser (2) au projecteur de lumière (3), et **en ce que** le chemin optique (r) est guidé vers le détecteur de lumière (60) par l'intermédiaire dudit au moins un miroir (50) du système de balayage (5).

6. Système de traitement laser (1) selon l'une quelconque des revendications 1 à 5, **caractérisé en ce que** le système de surveillance (6) est conçu pour effectuer une surveillance en profondeur dans une zone de surveillance (m) s'étendant dans la direction du chemin optique (r).

7. Système de traitement laser (1) selon l'une quelconque des revendications 1 à 6, **caractérisé en ce que** le système de surveillance (6) comprend au moins l'un : d'un système de détection interférométrique, d'un système de détection basé sur l'aberration chromatique axiale avec analyse spectrale, d'un système de détection confocal et d'un système de détection à triangulation.

8. Système de traitement laser (1) selon l'une quelconque des revendications 1 à 7, **caractérisé en ce que** le détecteur de lumière (60) comprend des éléments photosensibles qui sont conçus pour détecter au moins l'une de valeurs de luminosité et de plages spectrales.

9. Système de traitement laser (1) selon l'une quelconque des revendications 1 à 8, **caractérisé en ce que** le système de surveillance (6) comprend une source de lumière (63) destinée à éclairer la zone de surveillance (m).

10. Système de traitement laser (1) selon l'une quelconque des revendications 1 à 9, **caractérisé en ce que** le système de surveillance (6) est conçu pour surveiller, sur le chemin optique (r), une zone de surveillance (m) se déplaçant en association avec le foyer (F) et disposé en amont du foyer (F) dans la direction de déplacement du foyer (F) et non encore traitée par un rayonnement laser.

11. Système de traitement laser (1) selon l'une quelconque des revendications 1 à 10, **caractérisé en ce que** le système de surveillance (6) est conçu pour surveiller une pluralité de zones de surveillance (m) se déplaçant en association avec le foyer (F) sur plusieurs chemins optiques (r) différents qui interceptent une surface focale (f) passant par le foyer (F) respectivement en un autre point d'intersection (S) associé géométriquement de manière rigide au foyer (F).

12. Système de traitement laser (1) selon l'une quelconque des revendications 1 à 11, **caractérisé en ce que** le système de traitement laser (1) comprend une mémoire de données (62) contenant des données géométriques de structures oculaires détectées avant le traitement, et **en ce que** le système de surveillance (6) comprend une unité de traitement (62) couplée au détecteur de lumière (60), qui est conçue pour détecter des variations spatiales des structures oculaires définies par la géométrie en fonction des données géométriques et de signaux de détection du détecteur de lumière (60) dans la zone de surveillance (m).

13. Système de traitement laser (1) selon la revendication 12, **caractérisé en ce que** l'unité de traitement (62) est conçue pour générer des signaux de commande destinés au système de traitement laser (1) lors d'une détection de variations, dans lequel les signaux de commande comprennent au moins une instruction parmi les suivantes : la désactivation de la source laser (2) et une interruption de la projection du rayonnement laser (L).

14. Système de traitement laser (1) selon l'une quelconque des revendications 1 à 13, **caractérisé en ce que** le système de surveillance (6) est conçu pour surveiller, sur le chemin optique (r), une zone de surveillance (m) qui se déplace en association avec le foyer (F) de manière à ce qu'elle soit associée géométriquement de manière rigide au foyer (F).

15. Système de traitement laser (1) selon l'une quelconque des revendications 1 à 14, **caractérisé en ce que** le système de surveillance (6) comprend un système de focalisation (65) couplé dans le chemin optique (r) et disposé en amont du détecteur de lumière (60).
